# EUROPEAN PATENT APPLICATION

(11) **EP 3 995 003 A1**
(43) Date of publication of application: **11.05.2022**
(21) Application number: 20306319.3
(22) Date of filing: 04.11.2020
(51) Int. Cl.: A23L 33/15, A23L 33/16, A61K 31/122, A61K 31/19, A61K 31/4415, A61K 31/525, A61K 31/714, A61K 33/06, A61K 33/30, A61P 1/00, A61P 3/02, A61P 43/00

(54) **COMPOSITIONS FOR TREATING MINERALS AND VITAMINS DEFICIENCIES IN SLEEVE GASTRECTOMY PATIENTS**

(71) Applicant: Centre Hospitalier Universitaire de Nice, 06000 Nice (FR); Université Côte d'Azur, 06100 Nice (FR)
(72) Inventor: IANNELLI, Antonio, 06000 NICE (FR); SCHIAVO, Luigi, 81030 LUSCIANO (IT)
(74) Representative: Cabinet Becker et Associés

(57) **Abstract**

The present invention relates to nutrition compositions for use for preventing or treating minerals and vitamins deficiencies, in a patient who will undergo or has undergone sleeve gastrectomy, while avoiding the risks associated to inappropriate supplementation. Typically, the herein described compositions do not cause any micronutrient deficiencies or on the contrary absorption in excess of such micronutrients responsible for disorders such as for example hypo- and/or hypervitaminoses.

## Description

### FIELD OF THE INVENTION

The field of the present invention is that of medicine and human nutrition, in particular obese patient nutrition. The invention relates to nutrition compositions for use for preventing or treating minerals and vitamins deficiencies, in a patient who will undergo or has undergone sleeve gastrectomy, while avoiding the risks associated to inappropriate supplementation. Typically, the herein described compositions do not cause any micronutrient deficiencies or on the contrary absorption in excess of such micronutrients responsible for disorders such as for example hypo- and/or hypervitaminoses.
Inventors herein describe nutritional compositions in adequation with the particular needs of an obese patient around the specific weight-loss surgery called sleeve gastrectomy.

### PRIOR ART

Obesity and overweight are defined by the World Health Organization as abnormal or excessive fat accumulation that presents a risk to health. A crude population measure of obesity is the body mass index (BMI), a person's weight (in kilograms) divided by the square of his or her height (in meters). A person with a BMI of 30 or more is generally considered obese. A person with a BMI equal to or more than 25 is considered overweight. Obesity and overweight are major risk factors for a number of chronic diseases, including diabetes, cardiovascular diseases and cancer (herein identified as "derived or associated chronic diseases"). Once considered a problem only in high income countries, overweight and obesity are now dramatically on the rise in low- and middle-income countries, particularly in urban settings.
The goal of obesity treatment is for the patient to reach and stay at a healthy weight in order to improve overall health and lowers the risk of developing complications related to obesity. Reducing calories and practicing healthier eating habits are vital to overcoming obesity.
In some people, weight-loss surgery, also called bariatric surgery, is an option. Weight-loss surgery limits the amount of food the patient is able to comfortably eat or decreases the absorption of food and calories, or it does both. While weight-loss surgery offers the best chance of losing the most weight, it can pose serious risks. Weight-loss surgery for obesity is considered when other methods to lose weight haven't worked and typically for patients having extreme obesity (BMI of 40 or higher) or for patient having a BMI of 35 to 39.9 together with one or more obesity-related comorbidities such as diabetes, blood hypertension, sleep apnea syndrome or invalidating arthritis. Weight-loss surgery helps some people lose as much as 60% or more of their excess body weight, but weight-loss surgery isn't a miracle obesity cure. It doesn't guarantee that the patient will lose all of excess weight or that the patient will keep it off long term. Weight-loss success after surgery depends on patient's commitment to making lifelong changes in his/her eating and exercise habits and to select the good habits.
As reported in the American Society for Metabolic and Bariatric Surgery (ASMBS) guidelines, bariatric surgical procedures cause weight loss by restricting the amount of food the stomach can hold, causing malabsorption of nutrients, or by a combination of both gastric restriction and malabsorption. Bariatric procedures also often cause hormonal changes.
Most weight loss surgeries today are performed using minimally invasive techniques (laparoscopic surgery). The most common bariatric surgery procedures are sleeve gastrectomy followed by gastric bypass (also called "Roux-en-Y Gastric Bypass"). The adjustable gastric band is less performed nowadays while the biliopancreatic diversion with duodenal switch is rarely performed and only in tertiary referral centers for bariatric surgery. Each surgery has its own advantages and disadvantages.
In gastric bypass (Roux-en-Y gastric bypass), the surgeon creates a small pouch at the top of the stomach. The small intestine is then cut at a variable distance from the duodenum (around 50 cm); a Roux-en-Y loop is built (from 75 to 150 cm) and connected to the new pouch. Food and liquid flow directly from the pouch into this part of the intestine, bypassing most the stomach, the duodenum and part of the small bowel (from 1 meter to 2 meters generally).
In adjustable gastric banding, the stomach is separated into two pouches with an inflatable band. Pulling the band tight, like a belt, the surgeon creates a tiny channel between the two pouches. The band keeps the opening from expanding and is generally designed to stay in place permanently.
Biliopancreatic diversion with duodenal switch begins with the surgeon removing a large part of the stomach, doing a "sleeve gastrectomy" (see below). The surgeon then divides the duodenum after the pylorus (the sphincter that regulates the emptying of the stomach. A long Roux-en-Y loop is then built that bypasses most of the small bowel leaving only one meter of distal small bowel for absorption of nutrients. Bile and pancreatic juices join the ingested nutrients only in this part of the intestine where the absorption is then possible.

In sleeve gastrectomy, part of the stomach is removed, transforming the large reservoir that is the stomach in a narrow tube (or pipe). It's a less complicated surgery than gastric bypass or biliopancreatic diversion with duodenal switch.
In most bariatric procedures, the newly created stomach pouch is considerably smaller and facilitates significantly smaller meals, which translates into less calories consumed. Additionally, because there is less digestion of food by the smaller stomach pouch, and there is a segment of small intestine, in those procedures including an intestinal bypass, that would normally absorb calories as well as nutrients that no longer has food going through it, there is probably to some degree less absorption of calories and nutrients. Most importantly, the rerouting of the food stream produces changes in gut hormones that promote satiety, suppress hunger, and reverse one of the primary mechanisms by which obesity induces type 2 diabetes. Unfortunately, it's common to regain weight whatever the selected obesity treatment method performed. Patient might even regain weight after weight-loss surgery if he/she continues to overeat or overindulge in high-calorie foods or high-calorie beverages. The literature also suggests that bariatric surgery patients are at risk for deficiency of the following micronutrients after surgery: vitamins B12, B1, C, folate, A, D, and K, along with the trace minerals iron, selenium, zinc, and copper (Patel *et al.,* 2017). Indeed, Over-the-counter multivitamin and mineral supplements do not provide adequate amounts of certain nutrients such as vitamin B12, iron, or fat-soluble vitamins and patients will require additional doses of prophylactic supplementation life-long to maintain optimal micronutrient status (Schijns *et al.,* 2018).
As reported in the ASMBS guidelines, macro- and micronutrients assessment and dietary and micronutrients supplementation management is a crucial component of the BS process (Parrott *et al.,* 2017). The ASMBS document highlights the importance for the bariatric patient to regularly take vitamin and mineral supplements to prevent nutrient deficiencies that can worsen after surgery. However, there is no unanimity on the kind and amounts of multi-vitamins and multi-minerals supplementation that should be adopted after BS, and therefore supplementation practices vary widely. As consequence, often, the post-BS micronutrient prescriptions are unsatisfactorily based on expert opinion and personal experiences. In addition, currently, available over the counter multivitamins are either not specific for each procedure or when specifically formulated for each procedure they do not take into account the variable need in trace elements and vitamins following BS.
Deficiencies in vitamins and/or minerals can affect numerous metabolic functions such as the conversion of carbohydrates to energy, normal muscle function (including the heart muscle), oxidative carboxylation reactions, mitochondrial respiratory chain, coenzyme FAD and FMN formation, oxidation-reduction reactions in all cells, enzymatic reactions (mainly in protein and amino acid metabolisms), metabolism of fats and carbohydrates, Krebs cycle, synthesis of carnitine, catecholamines, adrenaline and noradrenaline, synthesis of cortisol, antioxidant, metabolic pathways involving cell growth, replication, and survival. These deficiencies can lead to numerous symptoms such as lack of energy, constipation, nausea, vomiting, depression, neuropathy, ataxia, confusion, Wernicke-Korsakoff Syndrome, decreased cardiovascular function, osteopenia, osteoporosis, skin problems, anemia, decreased learning capacity, insulin resistance, diarrhea, hypogonadism, neutropenia, bleeding, muscular cramping and arrhythmia. For example, osteomalacia is a deficiency disease due to calcium malabsorption (in which hypovitaminosis D occurs), renal loss of phosphorus or exceptionally an inhibition of crystal deposition on bone by aluminum or iron. Hypovitaminosis D may be also responsible for osteoporosis. Hypovitaminosis B12 may be responsible for pernicious anemia. A common disorder resulting from vitamin B1 deficiency is termed beriberi. Symptoms of this disorder range from vague fatigue, weakness, and confusion to difficulty walking upstairs, standing on one leg, and hallucination or psychosis. Vitamins B1 (thiamin) and B6 deficiencies can also lead to serious diseases such as Wernicke's encephalopathy. Hypervitaminosis A, D, E and/or K is a condition of abnormally high storage levels of vitamins, which can also lead to toxic symptoms. For example, hypervitaminosis A may lead to liver disorders from liver anomaly up to cirrhosis, and hypervitaminosis D may increase bone resorption and intestinal calcium absorption resulting in toxic hypercalcemia (the marked version thereof being often responsible for the symptoms).
Therefore, there is a need for all BS patients, and more particularly for SG patients, to receive not only a regular monitoring of serum micronutrient levels after surgery and periodically thereafter as recommended, but also, as herein taught by inventors to receive a micronutrients supplementation specifically formulated on the bases of the bariatric procedure performed and also the postoperative timing.
Inventors have developed and herein describe nutritional compositions containing specific micronutrients (vitamins and oligonutrients) in appropriate relative concentrations that vary as a function of the postoperative time and organism requirements in the context of sleeve gastrectomy (SG). As these advantageous compositions satisfy patient' needs, they can be used to prevent and treat vitamins and minerals deficiencies following SG.

### SUMMARY OF THE INVENTION

The invention is based on the demonstration by the inventors that a patient who has undergone sleeve gastrectomy (SG) needs vitamins and trace elements supplementation suitable for this particular surgery and for the postoperative period considered.

The object of the invention is a nutritional composition for use in prevention or treatment of micronutrient deficiencies of a human obese patient, typically human morbidly obese patient, who will undergo or has undergone sleeve gastrectomy, wherein the composition comprises vitamin A, vitamin B1, vitamin B6, vitamin B8 (biotin), vitamin B12, vitamin C, vitamin E, vitamin B9 (folate), vitamin D, in particular D3, iron bisglycinate or fumarate, magnesium citrate, selenium, zinc bisglycinate, copper gluconate or sulfate and, preferably, intrinsic factor.

The following advantageous particular nutritional compositions are herein described for the first time by inventors:
- a nutritional composition comprising, per unit dose, vitamin A in an amount consisting of about 8 000 IU to about 12 000 IU, vitamin B 1 in an amount consisting of about 10 mg to about 14 mg, vitamin B6 in an amount consisting of about 2.5 to about 3.5 mg, vitamin B8 (biotin) in an amount consisting of about 8 to about 10 mg, vitamin B12 in an amount consisting of about 450 to about 550 µg, vitamin C in an amount consisting of about 125 to about 175 mg, vitamin E in an amount consisting of about 12.5 to about 17.5 mg, vitamin B9 (folate) in an amount consisting of about 700 to about 900 µg, vitamin D3 in an amount consisting of about 1400 to about 1600 mg, iron bisglycinate or fumarate, preferably iron fumarate, in an amount consisting of about 17 to about 19 mg, magnesium citrate in an amount consisting of about 250 to about 350 mg, selenium in an amount consisting of about 50 to about 70 µg, zinc bisglycinate in an amount consisting of about 12.5 to about 17.5 mg, copper gluconate or sulfate, preferably copper gluconate, in an amount consisting of about 0.9 to about 1.1 mg, and intrinsic factor in an amount consisting of about 15 to about 25 mg, if the patient will undergo SG or has undergone SG at most four months ago, for example three months, two months or one month ago;
- a nutritional composition comprising, per unit dose, vitamin A in an amount consisting of about 4 500 IU to about 5 500 IU, vitamin B1 in an amount consisting of about 7 to about 9 mg, vitamin B6 in an amount consisting of about 1.5 to less than 2.5 mg, vitamin B8 (biotin) in an amount consisting of about 8 to about 10 mg, vitamin B12 in an amount consisting of about 350 to less than 450 µg, vitamin C in an amount consisting of about 125 to about 175 mg, vitamin E in an amount consisting of about 7.5 to about 12.5 mg, vitamin B9 (folate) in an amount consisting of about 500 to less than 700 µg, vitamin D3 in an amount consisting of about 1400 to about 1600 mg, iron bisglycinate or fumarate in an amount consisting of about 17 to about 19 mg, magnesium citrate in an amount consisting of about 150 to less than 250 mg, selenium in an amount consisting of about 30 to less than 50 µg, zinc bisglycinate in an amount consisting of about 7.5 to about 12.5 mg, copper gluconate or sulfate, preferably copper gluconate, in an amount consisting of about 0.6 to about less than 0.9 mg, and intrinsic factor in an amount consisting of about 15 to about 25 mg, if the patient has undergone SG four at most eight months ago, for example seven months, six months or five months ago;

- a nutritional composition comprising, per unit dose, vitamin A in an amount consisting of about 2 750 to about IU 4 250 IU, vitamin B 1 in an amount consisting of about 5 to less than 7 mg, vitamin B6 in an amount consisting of about 1 to less than 1.5 mg, vitamin B8 (biotin) in an amount consisting of about 4 to about 6 mg, vitamin B12 in an amount consisting of about 250 to less than 350 µg, vitamin C in an amount consisting of about 75 to less than 125 mg, vitamin E in an amount consisting of about 0.25 to about 0.75 mg, vitamin B9 (folate) in an amount consisting of about 300 to less than 500 µg, vitamin D3 in an amount consisting of about 1400 to about 1600 mg, iron bisglycinate or fumarate in an amount consisting of about 14 to about 16 mg, magnesium citrate in an amount consisting of about 50 to less than 150 mg, selenium in an amount consisting of about 10 to less than 30 µg, zinc bisglycinate in an amount consisting of about 7.5 to about 12.5 mg, copper gluconate or sulfate, preferably copper gluconate, in an amount consisting of about 0.4 to less than 0.6 mg, and intrinsic factor in an amount consisting of about 5 to less than 15 mg, if the patient has undergone SG more than eight, in particular more than 12 months ago;
- a nutritional composition comprising vitamin A in an amount consisting of about 10 000 IU, vitamin B1 in an amount consisting of about 12 mg, vitamin B6 in an amount consisting of about 3 mg, vitamin B8 (biotin) in an amount consisting of about 10 mg, vitamin B12 in an amount consisting of about 500 µg, vitamin C in an amount consisting of about 150 mg, vitamin E in an amount consisting of about 15 mg, vitamin B9 (folate) in an amount consisting of about 800 µg, vitamin D3 in an amount consisting of about 1500 mg, iron bisglycinate or fumarate in an amount consisting of about 18 mg, magnesium citrate in an amount consisting of about 300 mg, selenium in an amount consisting of about 60 µg, zinc bisglycinate in an amount consisting of about 15 mg, copper gluconate or sulfate, preferably copper gluconate, in an amount consisting of about 1 mg, and intrinsic factor in an amount consisting of about 20 mg;
- a nutritional composition comprising vitamin A in an amount consisting of about 5 000 IU, vitamin B 1 in an amount consisting of about 8 mg, vitamin B6 in an amount consisting of about 2 mg, vitamin B8 (biotin) in an amount consisting of about 10 mg, vitamin B12 in an amount consisting of about 400 µg, vitamin C in an amount consisting of about 150 mg, vitamin E in an amount consisting of about 10 mg, vitamin B9 (folate) in an amount consisting of about 600 µg, vitamin D3 in an amount consisting of about 1500 mg, iron bisglycinate or fumarate in an amount consisting of about 18 mg, magnesium citrate in an amount consisting of about 200 mg, selenium in an amount consisting of about 40 µg, zinc bisglycinate in an amount consisting of about 10 mg, copper gluconate or sulfate, preferably copper gluconate, in an amount consisting of about 0.8 mg, and intrinsic factor in an amount consisting of about 20 mg; and
- a nutritional composition comprising vitamin A in an amount consisting of about 4 000 IU, vitamin B 1 in an amount consisting of about 6 mg, vitamin B6 in an amount consisting of about 1.5 mg, vitamin B8 (biotin) in an amount consisting of about 5 mg, vitamin B12 in an amount consisting of about 300 µg, vitamin C in an amount consisting of about 100 mg, vitamin E in an amount consisting of about 0.5 mg, vitamin B9 (folate) in an amount consisting of about 400 µg, vitamin D3 in an amount consisting of about 1500 mg, iron bisglycinate or fumarate in an amount consisting of about 15 mg, magnesium citrate in an amount consisting of about 100 mg, selenium in an amount consisting of about 20 µg, zinc bisglycinate in an amount consisting of about 10 mg, copper gluconate or sulfate, preferably copper gluconate, in an amount consisting of about 0.5 mg, and intrinsic factor in an amount consisting of about 10 mg.

### DETAILED DESCRIPTION OF THE INVENTION

"BS" herein designates "Bariatric surgery" as well as "bariatric surgery/surgical procedure". "SG" herein designates "Sleeve gastrectomy", "laparoscopic sleeve gastrectomy", "sleeve" or "bariatric sleeve procedure" (which are equivalent terms for the skilled person).

Sleeve gastrectomy (SG) is incontestably the most performed bariatric procedure worldwide due to the several advantages it carries over other procedures including an intestinal bypass (Angrisani *et al.,* 2018). The main feature of the Sleeve gastrectomy (SG) procedure is the subtotal vertical gastrectomy. Approximately 80 percent of the stomach is removed. The remaining stomach is a tubular pouch that resembles a banana. SG works by several mechanisms. First, the new stomach pouch holds a considerably smaller volume than the normal stomach and helps to significantly reduce the amount of food (and thus calories) that can be consumed. The greater impact, however, seems to be the effect the surgery has on gut hormones that impact a number of factors including hunger, satiety, and blood sugar control.

Inventors now herein describe for the first time a composition providing balanced micronutrient supplementation, i.e. which will neither be responsible for an insufficient intake of vitamins and/or trace elements (minerals) nor for an excessive intake, both harmful for the patient. Herein described inventive compositions contain relative concentrations of vitamins and trace elements that vary as a function of (/are adapted to) the postoperative time and organism requirements in patients who will undergo or has undergone sleeve gastrectomy (SG). These compositions are for example in adequation with the patient's capacity to eat closer to a normal diet. Indeed, inventors have identified three phases including the 1 to 3 postoperative months, the 4 to 8 postoperative months, and the ninth (9^{th}) or twelfth (12^{th}) and following postoperative months. The composition the use of which is recommended during the last phase can be also used as a valuable nutritional composition (/micronutrient supplement) beyond 1 year because it contains a micronutrient amount compatible with the recommended dietary allowances (RDA) for the maintenance of a correct nutritional status because of the changes in food intake (both in terms of quality and amount).
The compositions according to the invention indeed have the significant advantage of being in line with the evolution of the amount of ingested food and, consequently, of vitamins and trace elements needs in the postoperative period. Indeed, patients' needs are not the same immediately after surgery and months or years later.
Herein described is a composition, in particular a nutritional composition for use in prevention or treatment of micronutrient deficiencies of a human obese patient who will undergo or has undergone sleeve gastrectomy (SG), wherein the composition comprises the following constituents: vitamin A (also commonly referred to as vitamin A acetate, retinyl palmitate, retinyl acetate, or its provitamin equivalent β-carotene & other carotenoids), vitamin B1 (thiamine and thiamine HC1), vitamin B6 (pyhdoxine, pyridoxine HC1, pyridoxal, and pyhdoxamine), vitamin B8 (biotin), vitamin B12 (cobalamin), vitamin C (ascorbic acid or sodium ascorbate), vitamin E [also commonly referred to as d-Alfa tocopheryl acid succinate (natural form), dl-alpha tocopheryl acid succinate (synthetic form - only d active)], vitamin B9 (folate and folic acid, also known as vitamin M), vitamin D (also commonly referred to as cholecalciferol or ergocalciferol), preferably vitamin D3 (cholecalciferol), iron bisglycinate or iron fumarate (preferably iron fumarate), magnesium citrate, selenium (in particular organic selenium), zinc bisglycinate, and copper gluconate or sulfate, preferably copper gluconate,, each constituent being in an adequate amount which depends on the postoperative period considered [between the three herein identified periods, i.e. 1) before SG or at most four months after, 2) between five and at most eight months after SG, and 3) more than eight or twelve months after SG] as herein below described.
Any one or more of the B vitamins may be in any of the numerous forms of the vitamins known in the art, including pharmaceutically acceptable salts thereof, chemically modified equivalents thereof, and mixtures thereof.
With respect to vitamin B 12 in particular, the cobalamin may be any of a number of known cobalamin compounds, including for example, cyanocobalamin, hydroxocobalamin (vitamin B12a), hydroxocobalamin HC1, sulfate, acetate and other hydroxocobalamin salts, aquacobalamin (vitamin B12b), nithlocobalamin (vitamin B12c), methylcobalamin (methyl B12), 5'-deoxyadenosine cobalamin (coenzyme B12), pharmaceutically acceptable salts thereof, chemically modified equivalents thereof, and mixtures thereof. Preferably, the cobalamin is cyanocobalamin.
The selection of the specific form of any one or more of the minerals to be used in the composition depends upon a number of factors known to those of skill in the art, including, for example, the composition in which the minerals are to be mixed or dissolved, the amount or concentration of the minerals desired in the composition, the solubility of the minerals, and the pH of the composition or resulting formulation thereof.

A particular and preferred nutritional composition for use in prevention or treatment of micronutrient deficiencies of a human obese patient who will undergo or has undergone sleeve gastrectomy (SG), and preferably in addition for use in prevention of obesity associated or derived chronic diseases, is a composition comprising vitamin A, vitamin B1, vitamin B6, vitamin B8 (biotin), vitamin B12, vitamin C, vitamin E, vitamin B9 (folate), vitamin D, preferably vitamin D3, iron bisglycinate or iron fumarate, preferably iron fumarate, magnesium citrate, selenium, zinc bisglycinate, copper gluconate or sulfate, preferably copper gluconate, and intrinsic factor.

Above identified constituents of the composition are well known to one skilled in the art as being suitable for human nutrition and available in the art.

In the context of the present invention, the expressions "unit dose", "dosage unit" or "dose unit" refer to an amount of a substance (or combination of substances) administered to an individual in a single dose. When administered in a single dose, any composition as herein described is thus to be considered as such a "unit dose", "dosage unit" or "dose unit". This amount is in particular and preferably the amount which is recommended "per day" for a patient who will undergo or has undergone sleeve gastrectomy (SG).
In particular aspects described herein below, the composition is administered via several "dosage units" distributed over the day, each dosage unit being for example administered with each meal (of which there are three to five per day).
In the context of the present invention, the patient is typically a human patient suffering, or who as suffered, of overweight, in particular of obesity, preferably of severe, extreme or morbid obesity, or is a subject having a BMI of 35 to 39.9 together with diabetes, blood hypertension, sleep apnea syndrome and/or joint arthritis.
As explained in the background part, a person with a body mass index (BMI) of 30 or more is generally considered obese. A person with a BMI equal to or more than 25 is considered overweight. Patients having severe/extreme/morbid obesity have a BMI of 40 or higher.
In the context of the present invention, a patient who will undergo SG is a patient who will undergo SG in the near future, typically in the next 6, 5, 4, 3 or 2 months or during the following month.
As used herein, the term "UI" or "IU" refers to a unit of measurement for the amount of a substance; the mass or volume that constitutes one international unit varies based on which substance is being measured, and the variance is based on the biological activity or effect, for the purpose of easier comparison across substances. International units are used to quantify vitamins, hormones, some medications, vaccines, blood products, and similar biologically active substances. Many biological agents exist in different forms or preparations (e.g. vitamin A in the form of retinol or beta-carotene). The goal of the IU is to be able to compare these, so that different forms or preparations with the same biological effect will contain the same number of IUs. To do so, the WHO Expert Committee on Biological Standardization provides a reference preparation of the agent, arbitrarily sets the number of IUs contained in that preparation, and specifies a biological procedure to compare other preparations of the same agent to the reference preparation. Since the number of IUs contained in a new substance is arbitrarily set, there is no equivalence between IU measurements of different biological agents. For instance, one IU of vitamin E cannot be equated with one IU of vitamin A in any way, including mass or efficacy. For example: vitamin A: 1 IU is the biological equivalent of 0.3 µg retinol, or of 0.6 µg beta-carotene; vitamin C: 1 IU is 50 µg L-ascorbic acid; vitamin D: 1 IU is the biological equivalent of 25 ng cholecalciferol/ergocalciferol; vitamin E: 1 IU is the biological equivalent of about 0.667 mg d-alpha-tocopherol (2/3 mg exactly), or of 0.45 mg of dl-alpha-tocopherol acetate.

The compositions herein described typically comprise vitamin A in an amount consisting of about 4000 to about 10 000 IU per unit dose.
The compositions herein described typically comprise vitamin B1 in an amount consisting of about 6 to about 12 mg per unit dose.
The compositions herein described typically comprise vitamin B6 in an amount consisting of about 1.5 to about 3 mg per unit dose.
The compositions herein described typically comprise vitamin B8 in an amount consisting of about 5 to about 10 mg per unit dose.
The compositions herein described typically comprise vitamin B12 in an amount consisting of about 300 to about 500 µg per unit dose
The compositions herein described typically comprise vitamin C in an amount consisting of about 100 to about 150 mg per unit dose.
The compositions herein described typically comprise vitamin E in an amount consisting of about 0.5 to about 15 mg per unit dose.
The compositions herein described typically comprise vitamin B9 in an amount consisting of about 400 to about 800 µg per unit dose.
The compositions herein described typically comprise vitamin D3 in an amount consisting of about 1400 to about 1600 mg per unit dose.
The compositions herein described typically comprise iron bisglycinate or fumarate, preferably iron fumarate, in an amount consisting of about 15 to about 18 mg per unit dose. A particular and preferred composition herein described comprises iron fumarate. Another particular composition herein described comprises iron bisglycinate.
The compositions herein described typically comprise magnesium citrate in an amount consisting of about 100 to about 300 mg per unit dose.
The compositions herein described typically comprise selenium in an amount consisting of about 20 to about 60 µg per unit dose.
The compositions herein described typically comprise zinc bisglycinate in an amount consisting of about 10 to about 15 mg per unit dose.
The compositions herein described typically comprise copper gluconate or sulfate in an amount consisting of about 0.5 to about 1 mg per unit dose. A particularly preferred composition herein described comprises copper gluconate. Another particular composition herein described comprises copper sulfate.
The compositions herein described preferably comprise intrinsic factor in an amount consisting of about 10 to about 20 mg per unit dose.

Particular compositions herein described comprise, per unit dose:
- vitamin A in an amount consisting of about 8 000 IU to about 12 000 IU, vitamin B1 in an amount consisting of about 10 mg to about 14 mg, vitamin B6 in an amount consisting of about 2.5 to about 3.5 mg, vitamin B8 (biotin) in an amount consisting of about 8 to about 10 mg, vitamin B12 in an amount consisting of about 450 to about 550 µg, vitamin C in an amount consisting of about 125 to about 175 mg, vitamin E in an amount consisting of about 12.5 to about 17.5 mg, vitamin B9 (folate) in an amount consisting of about 700 to about 900 µg, vitamin D3 in an amount consisting of about 1400 to about 1600 mg, iron bisglycinate or fumarate, preferably iron fumarate, in an amount consisting of about 17 to about 19 mg, magnesium citrate in an amount consisting of about 250 to about 350 mg, selenium in an amount consisting of about 50 to about 70 µg, zinc bisglycinate in an amount consisting of about 12.5 to about 17.5 mg, copper gluconate or sulfate, preferably copper gluconate, in an amount consisting of about 0.9 to about 1.1 mg, and, preferably, intrinsic factor in an amount consisting of about 15 to about 25 mg, if the patient will undergo SG or has undergone SG at most four months ago, for example three months, two months or one month ago;
- vitamin A in an amount consisting of about 4 500 IU to about 5 500 IU, vitamin B 1 in an amount consisting of about 7 to about 9 mg, vitamin B6 in an amount consisting of about 1.5 to less than 2.5 mg, vitamin B8 (biotin) in an amount consisting of about 8 to about 10 mg, vitamin B12 in an amount consisting of about 350 to less than 450 µg, vitamin C in an amount consisting of about 125 to about 175 mg, vitamin E in an amount consisting of about 7.5 to about 12.5 mg, vitamin B9 (folate) in an amount consisting of about 500 to less than 700 µg, vitamin D3 in an amount consisting of about 1400 to about 1600 mg, iron bisglycinate or fumarate, preferably iron fumarate, in an amount consisting of about 17 to about 19 mg, magnesium citrate in an amount consisting of about 150 to less than 250 mg, selenium in an amount consisting of about 30 to less than 50 µg, zinc bisglycinate in an amount consisting of about 7.5 to about 12.5 mg, copper gluconate or sulfate, preferably copper gluconate, in an amount consisting of about 0.6 to about less than 0.9 mg, and, preferably, intrinsic factor in an amount consisting of about 15 to about 25 mg, if the patient has undergone SG four at most eight months ago, for example seven months, six months or five months ago; and
- vitamin A in an amount consisting of about 2 750 to about IU 4 250 IU, vitamin B 1 in an amount consisting of about 5 to less than 7 mg, vitamin B6 in an amount consisting of about 1 to less than 1.5 mg, vitamin B8 (biotin) in an amount consisting of about 4 to about 6 mg, vitamin B12 in an amount consisting of about 250 to less than 350 µg, vitamin C in an amount consisting of about 75 to less than 125 mg, vitamin E in an amount consisting of about 0.25 to about 0.75 mg, vitamin B9 (folate) in an amount consisting of about 300 to less than 500 µg, vitamin D3 in an amount consisting of about 1400 to about 1600 mg, iron bisglycinate or fumarate, preferably iron fumarate, in an amount consisting of about 14 to about 16 mg, magnesium citrate in an amount consisting of about 50 to less than 150 mg, selenium in an amount consisting of about 10 to less than 30 µg, zinc bisglycinate in an amount consisting of about 7.5 to about 12.5 mg, copper gluconate or sulfate, preferably copper gluconate, in an amount consisting of about 0.4 to less than 0.6 mg, and, preferably, intrinsic factor in an amount consisting of about 5 to less than 15 mg, if the patient has undergone SG more than eight, in particular more than 12 months ago, for example more than 13, 14, 15, 16, 17, 18, 19, 20, 21, 22, 23, 24 months ago, or more than 3, 4, 5, 6, 7, 8, 9 or 10 years ago.

Preferably, the composition comprises, per unit dose:
- vitamin A in an amount consisting of about 10 000 IU, vitamin B 1 in an amount consisting of about 12 mg, vitamin B6 in an amount consisting of about 3 mg, vitamin B8 (biotin) in an amount consisting of about 10 mg, vitamin B12 in an amount consisting of about 500 µg, vitamin C in an amount consisting of about 150 mg, vitamin E in an amount consisting of about 15 mg, vitamin B9 (folate) in an amount consisting of about 800 µg, vitamin D3 in an amount consisting of about 1500 mg, iron bisglycinate or fumarate, preferably iron fumarate, in an amount consisting of about 18 mg, magnesium citrate in an amount consisting of about 300 mg, selenium in an amount consisting of about 60 µg, zinc bisglycinate in an amount consisting of about 15 mg, copper gluconate or sulfate, preferably copper gluconate, in an amount consisting of about 1 mg, and, preferably, intrinsic factor in an amount consisting of about 20 mg, if the patient will undergo SG or has undergone SG at most four months ago, for example three months, two months or one month ago;
- vitamin A in an amount consisting of about 5 000 IU, vitamin B1 in an amount consisting of about 8 mg, vitamin B6 in an amount consisting of about 2 mg, vitamin B8 (biotin) in an amount consisting of about 10 mg, vitamin B12 in an amount consisting of about 400 µg, vitamin C in an amount consisting of about 150 mg, vitamin E in an amount consisting of about 10 mg, vitamin B9 (folate) in an amount consisting of about 600 µg, vitamin D3 in an amount consisting of about 1500 mg, iron bisglycinate or fumarate, preferably iron fumarate, in an amount consisting of about 18 mg, magnesium citrate in an amount consisting of about 200 mg, selenium in an amount consisting of about 40 µg, zinc bisglycinate in an amount consisting of about 10 mg, copper gluconate or sulfate, preferably copper gluconate, in an amount consisting of about 0.8 mg, and, preferably, intrinsic factor in an amount consisting of about 20 mg, if the patient has undergone SG four at most eight months ago, for example seven months, six months or five months ago; and
- vitamin A in an amount consisting of about 4 000 IU, vitamin B1 in an amount consisting of about 6 mg, vitamin B6 in an amount consisting of about 1.5 mg, vitamin B8 (biotin) in an amount consisting of about 5 mg, vitamin B12 in an amount consisting of about 300 µg, vitamin C in an amount consisting of about 100 mg, vitamin E in an amount consisting of about 0.5 mg, vitamin B9 (folate) in an amount consisting of about 400 µg, vitamin D3 in an amount consisting of about 1500 mg, iron bisglycinate or fumarate, preferably iron fumarate, in an amount consisting of about 15 mg, magnesium citrate in an amount consisting of about 100 mg, selenium in an amount consisting of about 20 µg, zinc bisglycinate in an amount consisting of about 10 mg, copper gluconate or sulfate, preferably copper gluconate, in an amount consisting of about 0.5 mg, and, preferably, intrinsic factor in an amount consisting of about 10 mg, if the patient has undergone SG more than eight months, in particular more than 12 months ago, for example more than 13, 14, 15, 16, 17, 18, 19, 20, 21, 22, 23, 24 months ago, or more than 3, 4, 5, 6, 7, 8, 9 or 10 years ago.

A preferred nutritional composition (also herein identified as "Sleevit strong") comprises vitamin A in an amount consisting of about 10 000 IU, vitamin B1 in an amount consisting of about 12 mg, vitamin B6 in an amount consisting of about 3 mg, vitamin B8 (biotin) in an amount consisting of about 10 mg, vitamin B12 in an amount consisting of about 500 µg, vitamin C in an amount consisting of about 150 mg, vitamin E in an amount consisting of about 15 mg, vitamin B9 (folate) in an amount consisting of about 800 µg, vitamin D3 in an amount consisting of about 1500 mg, iron bisglycinate or fumarate, preferably iron fumarate, in an amount consisting of about 18 mg, magnesium citrate in an amount consisting of about 300 mg, selenium in an amount consisting of about 60 µg, zinc bisglycinate in an amount consisting of about 15 mg, copper gluconate or sulfate, preferably copper gluconate, in an amount consisting of about 1 mg, and intrinsic factor in an amount consisting of about 20 mg. This composition is preferably for use in a (human) patient as herein defined who will undergo SG or who has undergone SG at most four months ago, for example three months, two months or one month ago.

Another preferred nutritional composition (also herein identified as "Sleevit medium") comprises vitamin A in an amount consisting of about 5 000 IU, vitamin B1 in an amount consisting of about 8 mg, vitamin B6 in an amount consisting of about 2 mg, vitamin B8 (biotin) in an amount consisting of about 10 mg, vitamin B 12 in an amount consisting of about 400 µg, vitamin C in an amount consisting of about 150 mg, vitamin E in an amount consisting of about 10 mg, vitamin B9 (folate) in an amount consisting of about 600 µg, vitamin D3 in an amount consisting of about 1500 mg, iron bisglycinate or fumarate, preferably iron fumarate, in an amount consisting of about 18 mg, magnesium citrate in an amount consisting of about 200 mg, selenium in an amount consisting of about 40 µg, zinc bisglycinate in an amount consisting of about 10 mg, copper gluconate or sulfate, preferably copper gluconate, in an amount consisting of about 0.8 mg, and intrinsic factor in an amount consisting of about 20 mg. This composition is preferably for use in a (human) patient who has undergone SG four at most eight months ago, for example seven months, six months or five months ago.

Again another preferred nutritional composition (also herein identified as "Sleevit normal") comprises vitamin A in an amount consisting of about 4 000 IU, vitamin B1 in an amount consisting of about 6 mg, vitamin B6 in an amount consisting of about 1.5 mg, vitamin B8 (biotin) in an amount consisting of about 5 mg, vitamin B12 in an amount consisting of about 300 µg, vitamin C in an amount consisting of about 100 mg, vitamin E in an amount consisting of about 0.5 mg, vitamin B9 (folate) in an amount consisting of about 400 µg, vitamin D3 in an amount consisting of about 1500 mg, iron bisglycinate or fumarate, preferably iron fumarate, in an amount consisting of about 15 mg, magnesium citrate in an amount consisting of about 100 mg, selenium in an amount consisting of about 20 µg, zinc bisglycinate in an amount consisting of about 10 mg, copper gluconate or sulfate, preferably copper gluconate, in an amount consisting of about 0.5 mg, and intrinsic factor in an amount consisting of about 10 mg. This composition is preferably for use in a (human) patient who has undergone SG more than eight months, in particular more than 12 months ago, for example more than 13, 14, 15, 16, 17, 18, 19, 20, 21, 22, 23, 24 months ago, or more than 3, 4, 5, 6, 7, 8, 9 or 10 years ago.

In a particular and preferred aspect, the nutritional composition of the present technology is administered once daily.

In another particular aspect, the nutritional composition of the present technology is administered twice daily (i.e., two times per day).
In another particular aspect, the nutritional composition of the present technology is administered three times daily (i.e., three times per day).
In a further particular aspect, the nutritional composition is administered more than once daily, the time between administrations being at least 3 hours, at least 4 hours, at least 5 hours, at least 6 hours, at least 7 hours or at least 8 hours. For example, in the aspect wherein the nutritional composition is administered twice daily, the first dose may be administered in the morning whereas the second dose is administered at mid-day or in the evening.

The present description also relates to nutritional kits, each nutritional kit (also herein identified as a "daily kit") comprising several dosage units, for example two, three, four, five, six, seven, 15, 30 or 31 dosage units, the complete nutritional kit covering the needs of the patient per day, week or month(s), for example from one to six months, preferably along with instructions for taking the dosage units. As further explained herein below, a typical dosage unit is a tablet, a pill, a capsule, a caplet, or a powder. Preferably a dosage unit covers the needs of the patient per day.
A particular complete nutritional kit comprises for example dosage units in an amount sufficient to cover the needs of a patient for one, two, three or four weeks, or for two, three, four, five or six months.
Inventors in particular herein describe three distinct nutritional kits, each of them being adapted to one of the three herein identified distinct periods and for each day of a given period: 1) before SG or at most four months after, 2) between five and at most eight months after SG, and 3) more than eight or twelve months after SG.
A first nutritional kit, adapted for the first period which is before SG or at most four months after, comprises seven dosage units (one for each day of a week), two times (2 x) seven dosage units (to satisfy the need of the patient for two weeks), four times (4 x) seven dosage units (to satisfy the need of the patient for a month), eight times (8 x) seven dosage units (to satisfy the need of the patient for two months), twelve times (12 x) seven dosage units (to satisfy the need of the patient for three months), sixteen times (16 x) seven dosage units (to satisfy the need of the patient for four months) or twenty times (20 x) seven dosage units to satisfy the need of the patient for five months, for example for the period of time corresponding to the first month before SG and the four months after SG.

A second nutritional kit, adapted for the second period which is between five and at most eight months after SG, comprises seven dosage units (one for each day of a week), two times (2 x) seven dosage units (to satisfy the need of the patient for two weeks), four times (4 x) seven dosage units (to satisfy the need of the patient for a month), eight times (8 x) seven dosage units (to satisfy the need of the patient for two months), twelve times (12 x) seven dosage units (to satisfy the need of the patient for three months), or sixteen times (16 x) seven dosage units to satisfy the need of the patient for four months, for example for the period of time corresponding to five to eight months following SG.
A third nutritional kit, adapted for the third period which is more than eight or twelve months after SG, comprises seven dosage units (one for each day of a week), two times (2 x) seven dosage units (to satisfy the need of the patient for two weeks), four times (4 x) seven dosage units (to satisfy the need of the patient for a month), eight times (8 x) seven dosage units (to satisfy the need of the patient for two months), twelve times (12 x) seven dosage units (to satisfy the need of the patient for three months), sixteen times (16 x) seven dosage units (to satisfy the need of the patient for four months), or twenty times (20 x) seven dosage units, for example for the period of time corresponding to eight to twelve months following SG.

Other herein described kits comprise from about 7 days to about a month, two months, three months or four months supply of dosage units or "daily kits" as herein above described.

The compositions and dosage unit herein described may also comprise inactive components such as for example carriers, excipients, fillers or binders, disintegrating agents, lubricating agents, silica flow conditioners and stabilizing agents or the like, which may impart suitable or desirable characteristics to the dosage form such as taste, texture, viscosity, or the like. Disintegrating agents may assist in the dissolution of the tablet or pill. Disintegrating agents are well known in the art and include, but are not limited to alginic acid, carboxymethylcellulose, carboxymethylcellulose sodium, hydroxypropylcellulose (low substituted), microcrystalline cellulose, powdered cellulose, colloidal silicon dioxide, sodium croscarmellose, crospovidone, methylcellulose, polacrilin potassium, povidone, sodium alginate, sodium starch glycolate, starch, disodium di sulfite, disodium edathamil, disodium edetate, disodiumethylenediaminetetraacetate (EDTA), crosslinked polyvinylpyrollidines, pregelatanized starch, carboxymethyl starch, sodium carboxymethylstarch, microcrystalline cellulose. Lubricating agents assist in the compression of the formulation. Lubricating agents are well known in the art and include, but are not limited to calcium stearate, canola oil, glyceryl palmitosstearate, hydrogenated vegetable oil (type I), magnesium oxide, magnesium stearate, mineral oil, poloxamer, polyethylene glycols, sodium lauryl sulfate, sodium stearate fumarate, stearic acid, talc, zinc stearate, glyceryl behapate, magnesium lauryl sulfate, boric acid, sodium benzoate, sodium acetate, sodium benzoatelsodium acetate (in combination) and D,L-leucine. Fillers or binders include, but are not limited to acacia, alginic acid, calcium phosphate (dibasic), carboxymethylcellulose, carboxymethylcellulose sodium, hydroxy ethylcellulose, hydroxypropylcellulose, hydroxypropylmethylcellulose, dextrin, dextrates, sucrose, tylose, pregelatinized starch, calcium sulfate, amylose, glycine, bentonite, maltose, sorbitol, ethylcellulose, disodium hydrogen phosphate, disodium phosphate, disodium pyrosulfite, polyvinyl alcohol, gelatin, glucose, guar gum, liquid glucose, compressible sugar, magnesium aluminum silicate, maltodextrin, polyethylene oxide, polymethacrylates, povidone, sodium alginate, microcrystalline cellulose, starch and zein. Many other pharmaceutically acceptable tableting agents such as fillers or binders, lubricating agents, disintegrating agents, silica flow conditioners and stabilizing agents known in the pharmaceutical arts may be used in the multi-nutrients dosage units of the present technology (see, e.g. Remington: The Science and Practice of Pharmacy, 20th Edition, 2000, Lippincott Williams & Wilkins; Kibbe: Handbook of Pharmaceutical Excipients, 3rd Edition, 2000, American Pharmaceutical Association), incorporated herein by reference. As used herein, the expression "pharmaceutically acceptable" is any agent suitable for use in humans without undue side effects, such as irritation, toxicity, or allergic response.

Any of the herein described compositions of the invention can be formulated as a solid or as a liquid. Herein described compositions are preferably in a form suitable for oral administration. In a particular aspect, the composition or dosage unit is in a solid form. For instances, the composition is (in the form of) a tablet, a pill, a capsule, a caplet, or a powder, for example a flowable powder. Solid compositions may vary in shape and may be, for example, round, ovoid, oblong, cylindrical (e.g., disk-shaped) or any other geometric shape, for example rectilinear. For example, the composition can have a disk or ovoid shape, or a shape like a flattened disk or torpedo. The edges can be beveled or rounded.
When desired, the composition can be formulated with enteric coatings adapted for sustained or controlled release administration of the dosage form.
In a particular aspect, the composition is in a liquid form. In some aspects, the liquid form is suitable for oral administration. Liquid forms suitable for oral administration include liquid form preparations including emulsions, syrups, elixirs, aqueous solutions, aqueous suspensions, or solid form preparations which are intended to be converted shortly before use to liquid form preparations. Emulsions can be prepared in solutions, for example, in aqueous propylene glycol solutions or may contain emulsifying agents, for example, such as lecithin, sorbitan monooleate, or acacia. Aqueous solutions can be prepared by dissolving the alkaline mineral of the present technology in water and adding suitable colorants, flavors, stabilizers, and thickening agents. Aqueous suspensions can be prepared by dispersing the finely divided components in water with viscous material, such as natural or synthetic gums, resins, methylcellulose, sodium carboxymethylcellulose, and other well-known suspending agents. Solid form preparations include solutions, suspensions, and emulsions, and can comprise, in addition to the alkaline minerals of the present technology, colorants, flavors, stabilizers, buffers, artificial and natural sweeteners, dispersants, thickeners, solubilizing agents, and the like. Preferably the volume of a dosage unit should not exceed 150 ml, more preferably no more than 125 ml and even more preferably less than 100 ml. In some instances, the volume of a single dose is between about 5 ml and 25 ml.
The composition or dosage unit of the invention may further advantageously be a pasteurized (or sterilized) composition or dosage unit.

The compositions and dosage units of the present technology may be prepared by any of the methods well known in the art. Techniques and formulations generally are found in for example, Remington's Pharmaceutical Sciences (Mack Publishing Co., Easton, Pa.). Such methods include the step of bringing into an association of one or more ingredients with any inactive components. In general, the dosage forms are prepared by uniformly and intimately bringing into association the ingredients with liquid carriers or finely divided solid carriers or both, and then, if necessary, shaping the product or filling capsules. Methods of preparation of tablets are well known to one of ordinary skill in the art. See, e.g., Pharmaceutical Dosage Forms: Tablets, Third Edition, by Larry L. Augsburger and Stephen W. Hoag (publisher: Informa Healthcare; Dec. 15, 2007). These methods include direct compression and granulation (e.g., wet or dry or fluid-bed). The pellets can be made by, for example, simple granulation such as wet granulation or dry granulation, followed by sieving; extrusion and marumerization (spheronization); rotogranulation; or any agglomeration process that results in a pellet of reasonable size and robustness. For extrusion and marumerization, the ingredients are granulated by addition of a binder solution. The wet mass is passed through an extruder equipped with a certain size screen, and the extrudates are spheronized in a marumerizer. The resulting pellets are dried and sieved for further applications. One may also use high-shear granulation, wherein ingredients are dry-mixed and then the mixture is wetted by addition of a binder solution in a high shear-granulator/mixer. The granules are kneaded after wetting by the combined actions of mixing and milling. The resulting granules or pellets are dried and sieved for further applications. Alternatively, the immediate release beads or pellets are prepared by solution or suspension layering, whereby a solution or dispersion of the ingredients, with or without a binder and optionally an anti-tacking agent such as talc, is sprayed onto a core or starting seed (either prepared or a commercially available product) in a fluid bed processor or other suitable equipment. The cores or starting seeds can be, for example, sugar spheres or spheres made from microcrystalline cellulose. The ingredients, thus, are coated on the surface of the starting seeds. The ingredients may also be layered onto the ingredients-containing pellets described above, if desired. Following drug layering, the resulting ingredients-loaded pellets are dried for further applications. A protective layer, or overcoating, may be desired to ensure that the ingredients-loaded pellets do not aggregate during processing or upon storage. The protective coating layer may be applied immediately outside the core, either an ingredients-containing core or an ingredients-layered core, by conventional coating techniques such as pan coating or fluid bed coating using solutions of polymers in water or suitable organic solvents or by using aqueous polymer dispersions. Different anhydride-based polymers may be used as protective layer. In certain embodiments, many ingredients can be incorporated into the overcoating formula, for example to provide a quicker immediate release, such as plasticizers: acetyltriethyl citrate, triethyl citrate, acetyltributyl citrate; dibutylsebacate, triacetin, polyethylene glycols, propylene glycol and the others; lubricants: talc, colloidal silica dioxide, magnesium stearate, calcium stearate, titanium dioxide, magnesium silicate, and the like.
In yet other implementation, the compositions or dosage unit is in capsule form. Diverse capsule manufacturing and design methods are well known to one of ordinary skill in the art. See, e.g., Pharmaceutical Preformulation and Formulation: A Practical Guide from Candidate Drug Selection to Commercial Dosage Form, by Mark Gibson (publishers: Informa Healthcare, Aug. 1, 2001). When the dose form is a capsule, the method further comprises preparing the formulations into a form for loading and/or delivery, e.g., as a tablet, capsule and/or powder, and loading the formulations into the capsule to form the composition or unit dose.

In the context of the present invention, the composition is typically administered orally or via the enteral route. They are preferentially administered orally for the patient to optimize absorption of the constituents of the composition.

Also herein described is a method for providing nutritional supplementation to a patient as herein described, typically a human obese patient who will undergo or has undergone sleeve gastrectomy (SG), using one of the herein described composition the selection of which is to be adapted to the delay separating the patient from the sleeve gastrectomy. Such a method typically comprises a step of administering the patient with the selected proper composition if the patient will undergo SG or has undergone SG at most four months ago, if the patient has undergone SG four at most eight months ago, or if the patient has undergone SG more than eight, in particular more than 12 months ago. This is for maintaining necessary vitamins and minerals required levels in the patient's blood.

The claimed invention is illustrated in a non-limiting way by the figures and examples below. Identification of equivalent compositions, methods and kits are well within the skill of the ordinary practitioner and would require no more than routine experimentation, in light of the teachings of the present disclosure.

### FIGURES

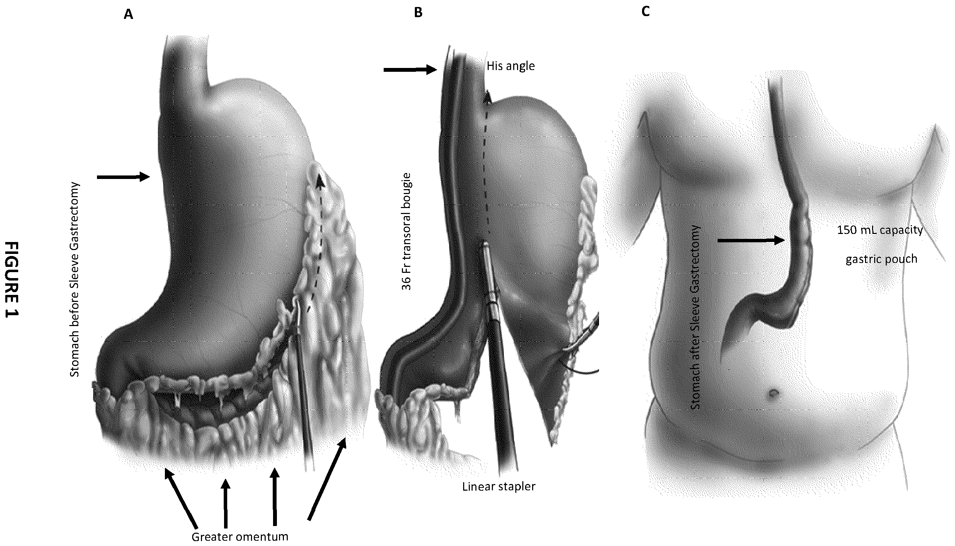

**Fig. 1****: Main steps of the sleeve gastrectomy procedure.**
   **(A)** The skeletisation of the great curvature of the stomach starts at 6 cm from the pylorus up to the angle of His.
   **(B)** A 36 Fr transoral bougie is positioned from the hiatus to the duodenum long the lesser curvature and the sleeve gastrectomy is performed using a linear stapler with different cartridges (green, blue and gold).
   **(C)** A 150 mL capacity gastric pouch is finally obtained.

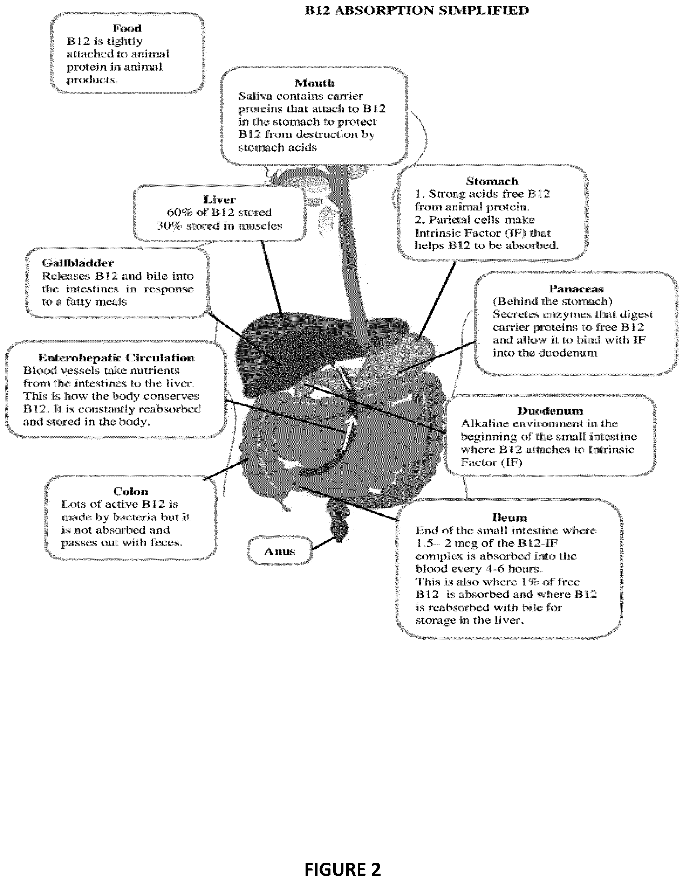

**Fig. 2****: General aspects of vitamin B12 absorption.**

### EXEMPLE

### Introduction

Bariatric surgery (BS) is the most effective long-term solution for the treatment and control of morbid obesity. In this context, sleeve gastrectomy (SG) represents one of the most performed bariatric procedures worldwide. Considerable attention is given to immediate surgical complications, including bleeding, staple-line leak, and stricture, however, the late consequences of SG anatomy, such as micronutrient deficiency (MD), are also of utmost importance. Certain micronutrients, mostly vitamin B12, vitamin D, folate, and iron, are the most common MD observed after SG. It is important to emphasize that there has been no unanimity on the appropriate type and amount of multi-vitamin and multi-mineral supplementation that should be prescribed after SG, and therefore supplementation practices vary widely. Often, the post-SG micronutrient prescriptions are based on expert opinion and personal experiences. Furthermore, to date, the micronutrient supplements prescribed after SG are characterized by having a single dosage (which is the same both in patients after 1 week of SG and in patients operated for 6 months or 1 year) and therefore do not conform to the evolution of the patient's diet which clearly changes over time.

### Patients

Herein, inventors report some data (not yet published) regarding the micronutrient status of a cohort of 74 patient (58 females, 16 males) who did not followed the prescribed post-SG micronutrient supplementation and they also show variations in the MD along the time.

### Biochemical assays

At the time of the study (3, 6 and 12 months after SG) Vitamin B12, folate, and 25-vitamin D were measured using the Vitros ECI Immunodiagnostic system (Ortho Clinical Diagnostics, Inc, Rochester, New York, USA), whereas iron was measured using the VITROS^{®} 4600 Chemistry System (Ortho Clinical Diagnostics, Inc, Rochester, NY, USA). In all patients, blood was collected by venepuncture between 7:30 a.m. and 10:30 a.m. after an overnight fast. Serum was separated by centrifugation at 2000 × *g* for 15 min at 4°C. Aliquots were processed immediately. The analyses were performed in an approved laboratory with internal and external quality control using the reagents provided by the manufacturer and following the manufacturer's instructions. Data were compared with accepted clinical cut-off values.

### Results

3-months after SG, patients were mostly deficient in vitamin B 12 (36.5%, 27/74), vitamin D (16.2%, 12/74), folate (24.3%, 18/74), iron (33.8%, 25/74), and were anaemic (59.5%, 44/74). Furthermore, 6-months after SG, inventors observed an increase of the percentage of patients showing MD as follow: vitamin B12 (47.3%, 35/74), vitamin D (46%, 34/74), folate (37.8%, 28/74), iron (47.3%, 35/74), and anaemia (59.4%, 44/74).

Moreover, 1 year after SG several patients still showed MD as follow: vitamin B12 (43%, 32/74), vitamin D (36.5%, 27/74), folate (33.8%, 25/74), iron (44.6%, 33/74), and anaemia (59.4%, 44/74).

### Discussion

SG patients are at risk of MD due to numerous mechanisms, including reduced food intake, decreased hydrochloric acid and intrinsic factor secretion, postsurgical vomiting and nausea, poor food choice, food avoidance (probably due to intolerance), and the low patient's adherence in following the post-SG prescribed diet and micronutrient supplements. Micronutrients plays a vital role as factors and cofactors in numerous physiological processes of the human body (i.e. hunger, resting metabolic rate, nutrient digestion and adsorption, fatty acid and carbohydrate metabolism, gland function, nervous central system activities, and others). Because SG is a restrictive procedure (the main classical surgical steps are reported in Figure 1) and therefore lacks the malabsorptive component, the risk for developing micronutrient deficiencies after SG is often considered low and therefore frequently not tested.

However, the resection of the stomach fundus with a consequent decreased hydrochloric acid and intrinsic factor secretion (that plays a key role in the vitamin B12 adsorption, Figure 2) associated to reduced intake, postoperative nausea and vomiting, poor food choice, and food avoidance due to intolerance may render the patients prone to several vitamin and mineral deficiencies, and in turn more susceptible to develop several disorders especially anemia, hair loss, metabolic and neurological disorders. Therefore, following SG, multi-vitamin and multi-mineral supplementation is mandatory. Furthermore, because many MD progress with time, patients that undergo SG must be monitored regularly and properly supplemented by specific formulations to avoid short- and long-term MD
Although it is universally accepted that the best way to introduce in our body vitamins and minerals is through food rich sources after SG patients unfortunately often are low adherent in following the post-SG prescribed diet and, consequently, often fail to consume an appropriate amount of foods that are rich in vitamins and minerals (i.e. fruits, vegetables, legumes, and cereals). Inventors herein demonstrate that a daily multi-vitamin and multi-mineral supplement is essential after all bariatric procedures, even the purely restrictive procedures, such as SG.

### Conclusions

SG is extensively performed as BS procedure, but usually lead to micronutrient (vitamins and minerals) deficiencies. Therefore, preoperative life style modifications, micronutrient assessment and a rigorous postoperative follow-up plan with administration of multi-vitamin and multi-mineral supplements, together with a regular assessment of micronutrient serum levels, are life-long recommendation after all bariatric procedures, even for restrictive procedures such as SG.
Compositions herein described by inventors are advantageously in line with the evolution of amount of ingested food and, consequently, of vitamins and trace elements need in the postoperative period. Indeed, patients' needs are not the same immediately after surgery and months or years later. In addition, inventors herein describe for the first-time pre- as well as post-SG formulations containing the intrinsic factor. They believe that after SG, its presence in the post-operative micronutrient supplementation plays an important role in avoiding vitamin B12 deficiency.

## Claims

1. A nutritional composition for use in prevention or treatment of micronutrient deficiencies of a human obese patient who will undergo or has undergone sleeve gastrectomy (SG), wherein the composition comprises vitamin A, vitamin B1, vitamin B6, vitamin B8 (biotin), vitamin B12, vitamin C, vitamin E, vitamin B9 (folate), vitamin D, iron bisglycinate or fumarate, magnesium citrate, selenium, zinc bisglycinate, copper gluconate or sulfate and intrinsic factor.

2. The composition of claim 1, wherein the composition comprises vitamin A in an amount consisting of about 4000 to about 10 000 IU per unit dose.

3. The composition of claim 1, wherein the composition comprises vitamin B1 in an amount consisting of about 6 to about 12 mg per unit dose.

4. The composition of claim 1, wherein the composition comprises vitamin B6 in an amount consisting of about 1.5 to about 3 mg per unit dose.

5. The composition of claim 1, wherein the composition comprises vitamin B8 in an amount consisting of about 5 to about 10 mg per unit dose.

6. The composition of claim 1, wherein the composition comprises vitamin B12 in an amount consisting of about 300 to about 500 µg per unit dose.

7. The composition of claim 1, wherein the composition comprises vitamin D3 in an amount consisting of about 1400 to about 1600 mg per unit dose.

8. The composition of claim 1, wherein the composition comprises iron fumarate.

9. The composition of claim 1, wherein the composition comprises intrinsic factor in an amount consisting of about 10 to about 20 mg per unit dose.

10. The composition of claim 1, wherein the composition comprises, per unit dose:
- vitamin A in an amount consisting of about 8 000 IU to about 12 000 IU, vitamin B1 in an amount consisting of about 10 mg to about 14 mg, vitamin B6 in an amount consisting of about 2.5 to about 3.5 mg, vitamin B8 (biotin) in an amount consisting of about 8 to about 10 mg, vitamin B12 in an amount consisting of about 450 to about 550 µg, vitamin C in an amount consisting of about 125 to about 175 mg, vitamin E in an amount consisting of about 12.5 to about 17.5 mg, vitamin B9 (folate) in an amount consisting of about 700 to about 900 µg, vitamin D3 in an amount consisting of about 1400 to about 1600 mg, iron bisglycinate or fumarate in an amount consisting of about 17 to about 19 mg, magnesium citrate in an amount consisting of about 250 to about 350 mg, selenium in an amount consisting of about 50 to about 70 µg, zinc bisglycinate in an amount consisting of about 12.5 to about 17.5 mg, copper gluconate or sulfate in an amount consisting of about 0.9 to about 1.1 mg, and intrinsic factor in an amount consisting of about 15 to about 25 mg, if the patient will undergo SG or has undergone SG at most four months ago, for example three months, two months or one month ago;
- vitamin A in an amount consisting of about 4 500 IU to about 5 500 IU, vitamin B1 in an amount consisting of about 7 to about 9 mg, vitamin B6 in an amount consisting of about 1.5 to less than 2.5 mg, vitamin B8 (biotin) in an amount consisting of about 8 to about 10 mg, vitamin B12 in an amount consisting of about 350 to less than 450 µg, vitamin C in an amount consisting of about 125 to about 175 mg, vitamin E in an amount consisting of about 7.5 to about 12.5 mg, vitamin B9 (folate) in an amount consisting of about 500 to less than 700 µg, vitamin D3 in an amount consisting of about 1400 to about 1600 mg, iron bisglycinate or fumarate in an amount consisting of about 17 to about 19 mg, magnesium citrate in an amount consisting of about 150 to less than 250 mg, selenium in an amount consisting of about 30 to less than 50 µg, zinc bisglycinate in an amount consisting of about 7.5 to about 12.5 mg, copper gluconate or sulfate in an amount consisting of about 0.6 to about less than 0.9 mg, and intrinsic factor in an amount consisting of about 15 to about 25 mg, if the patient has undergone SG four at most eight months ago, for example seven months, six months or five months ago; and
- vitamin A in an amount consisting of about 2 750 to about IU 4 250 IU, vitamin B1 in an amount consisting of about 5 to less than 7 mg, vitamin B6 in an amount consisting of about 1 to less than 1.5 mg, vitamin B8 (biotin) in an amount consisting of about 4 to about 6 mg, vitamin B12 in an amount consisting of about 250 to less than 350 µg, vitamin C in an amount consisting of about 75 to less than 125 mg, vitamin E in an amount consisting of about 0.25 to about 0.75 mg, vitamin B9 (folate) in an amount consisting of about 300 to less than 500 µg, vitamin D3 in an amount consisting of about 1400 to about 1600 mg, iron bisglycinate or fumarate in an amount consisting of about 14 to about 16 mg, magnesium citrate in an amount consisting of about 50 to less than 150 mg, selenium in an amount consisting of about 10 to less than 30 µg, zinc bisglycinate in an amount consisting of about 7.5 to about 12.5 mg, copper gluconate or sulfate in an amount consisting of about 0.4 to less than 0.6 mg, and intrinsic factor in an amount consisting of about 5 to less than 15 mg, if the patient has undergone SG more than eight, in particular more than 12 months ago.

11. The composition of claim 10, wherein the composition comprises, per unit dose:
- vitamin A in an amount consisting of about 10 000 IU, vitamin B1 in an amount consisting of about 12 mg, vitamin B6 in an amount consisting of about 3 mg, vitamin B8 (biotin) in an amount consisting of about 10 mg, vitamin B12 in an amount consisting of about 500µg, vitamin C in an amount consisting of about 150 mg, vitamin E in an amount consisting of about 15 mg, vitamin B9 (folate) in an amount consisting of about 800 µg, vitamin D3 in an amount consisting of about 1500 mg, iron bisglycinate or fumarate in an amount consisting of about 18 mg, magnesium citrate in an amount consisting of about 300 mg, selenium in an amount consisting of about 60 µg, zinc bisglycinate in an amount consisting of about 15 mg, copper gluconate or sulfate in an amount consisting of about 1 mg, and intrinsic factor in an amount consisting of about 20 mg, if the patient will undergo SG or has undergone SG at most four months ago, for example three months, two months or one month ago;
- vitamin A in an amount consisting of about 5 000 IU, vitamin B1 in an amount consisting of about 8 mg, vitamin B6 in an amount consisting of about 2 mg, vitamin B8 (biotin) in an amount consisting of about 10 mg, vitamin B12 in an amount consisting of about 400 µg, vitamin C in an amount consisting of about 150 mg, vitamin E in an amount consisting of about 10 mg, vitamin B9 (folate) in an amount consisting of about 600 µg, vitamin D3 in an amount consisting of about 1500 mg, iron bisglycinate or fumarate in an amount consisting of about 18 mg, magnesium citrate in an amount consisting of about 200 mg, selenium in an amount consisting of about 40 µg, zinc bisglycinate in an amount consisting of about 10 mg, copper gluconate or sulfate in an amount consisting of about 0.8 mg, and intrinsic factor in an amount consisting of about 20 mg, if the patient has undergone SG four at most eight months ago, for example seven months, six months or five months ago; and
- vitamin A in an amount consisting of about 4 000 IU, vitamin B1 in an amount consisting of about 6 mg, vitamin B6 in an amount consisting of about 1.5 mg, vitamin B8 (biotin) in an amount consisting of about 5 mg, vitamin B12 in an amount consisting of about 300 µg, vitamin C in an amount consisting of about 100 mg, vitamin E in an amount consisting of about 0.5 mg, vitamin B9 (folate) in an amount consisting of about 400 µg, vitamin D3 in an amount consisting of about 1500 mg, iron bisglycinate or fumarate in an amount consisting of about 15 mg, magnesium citrate in an amount consisting of about 100 mg, selenium in an amount consisting of about 20 µg, zinc bisglycinate in an amount consisting of about 10 mg, copper gluconate or sulfate in an amount consisting of about 0.5 mg, and intrinsic factor in an amount consisting of about 10 mg, if the patient has undergone SG more than eight months, in particular more than 12 months ago.

12. The composition of anyone of claims 1 to 11, wherein the composition is formulated as a solid or as a liquid, for example as a tablet or pill.

13. A nutritional composition comprising vitamin A in an amount consisting of about 10 000 IU, vitamin B1 in an amount consisting of about 12 mg, vitamin B6 in an amount consisting of about 3 mg, vitamin B8 (biotin) in an amount consisting of about 10 mg, vitamin B12 in an amount consisting of about 500 µg, vitamin C in an amount consisting of about 150 mg, vitamin E in an amount consisting of about 15 mg, vitamin B9 (folate) in an amount consisting of about 800 µg, vitamin D3 in an amount consisting of about 1500 mg, iron bisglycinate or fumarate in an amount consisting of about 18 mg, magnesium citrate in an amount consisting of about 300 mg, selenium in an amount consisting of about 60 µg, zinc bisglycinate in an amount consisting of about 15 mg, copper gluconate or sulfate in an amount consisting of about 1 mg, and intrinsic factor in an amount consisting of about 20 mg.

14. A nutritional composition comprising vitamin A in an amount consisting of about 5 000 IU, vitamin B 1 in an amount consisting of about 8 mg, vitamin B6 in an amount consisting of about 2 mg, vitamin B8 (biotin) in an amount consisting of about 10 mg, vitamin B12 in an amount consisting of about 400 µg, vitamin C in an amount consisting of about 150 mg, vitamin E in an amount consisting of about 10 mg, vitamin B9 (folate) in an amount consisting of about 600 µg, vitamin D3 in an amount consisting of about 1500 mg, iron bisglycinate or fumarate in an amount consisting of about 18 mg, magnesium citrate in an amount consisting of about 200 mg, selenium in an amount consisting of about 40 µg, zinc bisglycinate in an amount consisting of about 10 mg, copper gluconate or sulfate in an amount consisting of about 0.8 mg, and intrinsic factor in an amount consisting of about 20 mg.

15. A nutritional composition comprising vitamin A in an amount consisting of about 4 000 IU, vitamin B 1 in an amount consisting of about 6 mg, vitamin B6 in an amount consisting of about 1.5 mg, vitamin B8 (biotin) in an amount consisting of about 5 mg, vitamin B12 in an amount consisting of about 300 µg, vitamin C in an amount consisting of about 100 mg, vitamin E in an amount consisting of about 0.5 mg, vitamin B9 (folate) in an amount consisting of about 400 µg, vitamin D3 in an amount consisting of about 1500 mg, iron bisglycinate or fumarate in an amount consisting of about 15 mg, magnesium citrate in an amount consisting of about 100 mg, selenium in an amount consisting of about 20 µg, zinc bisglycinate in an amount consisting of about 10 mg, copper gluconate or sulfate in an amount consisting of about 0.5 mg, and intrinsic factor in an amount consisting of about 10 mg.
